# EUROPÄISCHE PATENTSCHRIFT

(11) **EP 4 197 943 B1**
(45) Veröffentlichungstag und Bekanntmachung des Hinweises auf die Patenterteilung: **01.05.2024**
(21) Anmeldenummer: 22211340.9
(22) Anmeldetag: 05.12.2022
(51) Int. Cl.: B65G 43/00, B07C 5/34, B08B 9/46, B65G 47/91

(54) **VERFAHREN ZUM HANDHABEN VON LEERDOSENLAGEN UND VORRICHTUNG ZUM HANDHABEN VON LEERDOSENLAGEN**
METHOD FOR HANDLING EMPTY CAN LAYERS AND DEVICE FOR HANDLING EMPTY CAN LAYERS
PROCÉDÉ DE MANIPULATION DE COUCHES DE BOÎTES VIDES ET DISPOSITIF DE MANIPULATION DE COUCHES DE BOÎTES VIDES

(30) Priorität: 13.12.2021 DE 102021132817
(43) Veröffentlichungstag der Anmeldung: 21.06.2023
(73) Patentinhaber: Krones Aktiengesellschaft, 93073 Neutraubling (DE)
(72) Erfinder: ZEINER, Peter, 93073 Neutraubling (DE); KIRSCHNER, Peter, 93073 Neutraubling (DE); HENSEL, Thomas, 93073 Neutraubling (DE); PERL, Kurt, 93073 Neutraubling (DE)
(74) Vertreter: Benninger, Johannes

(56) Entgegenhaltungen:
- WO-A1-98/47795
- DE-A1- 19 505 474
- DE-U1-202017 104 004

## Beschreibung

Die vorliegende Erfindung betrifft ein Verfahren zum Handhaben von Leerdosenlagen sowie eine Vorrichtung zum Handhaben von Leerdosenlagen.

Insbesondere betrifft die vorliegende Erfindung ein Verfahren und eine Vorrichtung zum Ausschleusen defekter Leerdosen vor der weiteren Verarbeitung, beispielsweise vor der Befüllung und dem Verschließen derselben. Ein solches Verfahren und eine solche Vorrichtung findet insbesondere in der Getränkeindustrie Verwendung.

Bei der Herstellung von Getränkedosen o.ä. werden die hierfür verwendeten Leerdosen als Neubehälterdosen in Palettenlagen bereitgestellt. Die Neubehälterdosen sind insbesondere leere Leerdosen ohne oberen Deckel. Nachfolgend wird in diesem Zusammenhang jeweils von Leerdosen gesprochen und die Palettenlagen werden als Leerdosenlagen bezeichnet. In der Regel sind mehrere Lagen an Leerdosen übereinandergestapelt auf einer Palette angeordnet, wobei zwischen den übereinandergestapelten Leerdosenlagen jeweils eine Zwischenlage aus einem flächigen Material, beispielsweise eine Papierlage, eine dünne Kunststofflage, eine Karton-Zwischenlage o.ä. angeordnet sein kann.

Die Leerdosenlagen werden im Stand der Technik in der Regel allumfänglich von einer Greifeinrichtung umfasst und auf eine Transporteinrichtung übergeschoben, über welche Transporteinrichtung die Leerdosenlage der weiteren Verarbeitung zugeführt wird.

Probleme können insbesondere in solchen Fällen auftreten, wenn in der Leerdosenlage fehlerhafte Leerdosen enthalten sind. Ein Defekt einer fehlerhaften Leerdose kann beispielsweise auf einen Produktionsfehler, auf Beschädigungen beim Transport o.ä. Faktoren zurückzuführen sein. Der Defekt kann beispielsweise in Form von Verbiegungen, Dellen, Knicken, Schnitten o.ä. an den Leerdosen bestehen. Da defekte oder fehlerhafte Leerdosen vorzugsweise nicht verarbeitet werden sollen, da in diesem Fall defekte Produkte entstehen würden, ist es wünschenswert, diese rechtzeitig zu identifizieren und vor der weiteren Verarbeitung aus dem Verarbeitungsprozess auszuschleusen.

Die Patentschrift EP 0 040 661 B1 bezieht sich auf ein Verfahren zum automatischen Aussortieren defekter leerer Leerdosen sowie auf eine entsprechende Vorrichtung. Diese beinhalten einen Flanschabschnitt-Inspektionsprozess und einen Druckqualitäts-Inspektionsprozess und sind mit einem Leerdosen-Zuführmechanismus und/oder Leerdosen-Ausgabemechanismus ausgestattet, um eine kontinuierlich ankommende Gruppe leerer Leerdosen zu einer vorbestimmten Position in einer Inspektionsstation intermittierend einzeln zu befördern. Die einzeln in der Inspektionsstation der Vorrichtung positionierte Leerdose wird über einen Drehmechanismus gedreht. Während der Drehung wird über einen Magnetkopf eine Änderung eines statischen Magnetfelds detektiert, wodurch Fehler erkannt werden können.

Die Patentschrift EP 0 055 296 B1 bezieht sich auf einen rotierenden Lichttester zum Inspizieren leerer Leerdosen. Die Leerdosen werden hierbei nach unterschiedlichen Defekten untersucht, wobei die Leerdosen während der Untersuchung nicht beschädigt werden. Auf diese können Weise beispielsweise Risse in den Leerdosen identifiziert werden, welche zu einer Undichtigkeit der befüllten Leerdosen führen würden.

Die bekannten Verfahren sind in technisch sehr aufwändig und beinhalten einen nicht zu vernachlässigenden Zeitaufwand, da die Leerdosen jeweils einzeln überprüft werden.

DE202017104004U1 offenbart ein Verfahren gemäß dem Oberbegriff des Anspruchs 1 und eine Vorrichtung gemäß dem Oberbegriff des Anspruchs 9.

Die Aufgabe der Erfindung besteht darin, die Nachteile des bekannten Stands der Technik zu beseitigen und insbesondere ein einfaches Verfahren und eine entsprechende Vorrichtung bereitzustellen, welches es erlaubt, defekte Leerdosen in einfacher und zeitsparender Weise zu identifizieren und vor der Handhabung durch nachfolgende Verarbeitungseinrichtungen aus der laufenden Produktion auszuschleusen.

Die obige Aufgabe wird durch ein Verfahren und eine Vorrichtung zum Handhaben von Leerdosenlagen gelöst, die die Merkmale in den unabhängigen Patentansprüchen umfassen. Weitere vorteilhafte Ausgestaltungen werden durch die Unteransprüche beschrieben.

Eine Leerdosenlage umfasst eine Mehrzahl an regelmäßig angeordneten Leerdosen mit oberseitigen Öffnungen und seitlichen Mantelflächen.

Der Kern der Erfindung betrifft das Abwerfen und Entsorgen von fehlerhaften Leerdosen einer Leerdosenlage bzw. aus einer Leerdosenlage, so dass nur intakte Leerdosen der weiteren Verarbeitung, insbesondere einer Befüllung etc., zugeführt werden.

Dabei wird der Effekt ausgenutzt, dass sich intakte Leerdosen beim Anlegen des Unterdrucks mit dem vordefinierten Grenzwert nicht oder zumindest nur in elastischer Weise verformen. Dagegen werden defekte Leerdosen entweder plastisch verformt oder sie kollabieren, etwa durch ein Implodieren der defekten Leerdosen.

Wenn im vorliegenden Zusammenhang von einer Erhöhung des Unterdrucks gesprochen wird, so ist darunter zu verstehen, dass die Druckdifferenz zwischen dem Unterdruck und einem Normaldruck, insbesondere weiter erhöht wird. Unter Normaldruck ist insbesondere ein vereinbarter fester Gasdruck zu verstehen, der einem mittleren Luftdruck an der Erdoberfläche entspricht. Er beträgt 101 325 Pa = 1,013 25 bar.

Unter defekten oder fehlerhaften Leerdosen sind insbesondere solche zu verstehen, welche bereits eingeknickt oder eingedellt sind oder sonstige Verformungen aufweisen. Auch stellen Risse im Leerdosenmaterial einen Fehler dar, der zu fehlerhaften Produkten führen würde. Somit ist es auch notwendig, solche fehlerhaften Leerdosen 24 zu identifizieren und vor der weiteren Verarbeitung aus dem Produktionsprozess auszuschleusen. Hierbei wird insbesondere der Effekt ausgenutzt, dass auch Risse in Leerdosen dazu führen, dass es beim Anlegen eines auf einen Grenzwert erhöhten Unterdrucks zu einer Verformung der fehlerhaften Leerdosen kommt.

Die Leerdosenlagen werden an einem Aufnahmeplatz bereitgestellt. Insbesondere werden mehrere auf einer Palette übereinandergestapelte Leerdosenlagen an dem Aufnahmeplatz bereitgestellt.

Zum Erfassen der Leerdosenlage werden alle Leerdosen einer Leerdosenlage durch Anlegen eines Unterdrucks an den oberseitigen Öffnungen der Leerdosen angesaugt und saugend gehalten. Insbesondere werden alle Leerdosen einer Leerdosenlage gleichzeitig durch Anlegen eines Unterdrucks an den oberseitigen Öffnungen der Leerdosen angesaugt und saugend gehalten
Der saugende Unterdruck wird bis zu einem vordefinierten Grenzwert gesteigert. Der Grenzwert wird durch einen Unterdruck gebildet, bei welchem die auf die Mantelflächen der Leerdosen wirkenden Druckkräfte von intakten Leerdosen durch die Eigenstabilität zumindest soweit kompensiert werden, dass sich die Mantelflächen nach Aufheben oder Reduzieren des Unterdrucks elastisch in ihre Ausgangskontur zurückverformen. Der Grenzwert ist dabei abhängig von den jeweiligen Leerdosen und bestimmt sich beispielsweise aufgrund von deren Raumvolumen, dem Leerdosenmaterial, der Wandstärke und gegebenenfalls weiterer Parameter.

Insbesondere werden mechanisch intakte und/oder unverformte Leerdosen bis zum Grenzwert des Unterdrucks weiterhin saugend gehalten. Dagegen werden fehlerhafte Leerdosen vor oder bei Erreichen des Grenzwertes plastisch verformt. Insbesondere können fehlerhafte Leerdosen vor oder bei Erreichen des Grenzwertes implodieren.

Aufgrund der Verformung oder Implosion der fehlerhaften Leerdosen werden diese nicht mehr ausreichend saugend gehalten und Lösen sich aus der Leerdosenlage und insbesondere aus der saugenden Verbindung.

Eine Ausführungsform sieht vor, dass die saugend gehaltenen mechanisch intakten und/oder unverformten Leerdosen einem Ablageplatz und/oder der weiteren Verarbeitung zugeführt werden. Die fehlerhaften Leerdosen werden dagegen aus dem Produktionsbetrieb entfernt.

Eine weitere Ausführungsform sieht vor, dass die Leerdosenlage mit einem ersten Saugdruck angesaugt und saugend gehalten wird. Die saugend gehaltene Leerdosenlage wird oberhalb eines Auffangbehälters angeordnet. Nunmehr wird der saugende Unterdruck auf den Grenzwert gesteigert, wobei sich fehlerhafte Leerdosen aus der Leergutlage lösen und in den Auffangbehälter fallen. Anschließend werden die weiterhin saugend gehaltenen mechanisch intakten und/oder unverformten Leerdosen der weiteren Verarbeitung zugeführt.

Eine alternative Ausführungsform sieht vor, dass die Leerdosenlage angesaugt und saugend gehalten wird. Dabei kann bereits beim Ansaugen der Unterdruck direkt auf den Grenzwert eingestellt werden. Nunmehr wird die saugend gehaltene Leerdosenlage angehoben. Die fehlerhaften Leerdosen verbleiben hierbei am Aufnahmeplatz und können durch Einsatz geeigneter Einrichtungen, beispielsweise von einem Schiebeelement o.ä., aus dem Produktionsprozess entfernt werden.

Eine weitere Ausführungsform sieht vor, dass die saugend gehaltene Leerdosenlage nach Einstellung des Grenzwertes über einen Aussonderungs- oder Auffangbereich geführt wird, welcher Aussonderungs- oder Auffangbereich sich durch ein geringeres Höhenniveau auszeichnet, so dass fehlerhafte Leerdosen in den Aussonderungs- oder Auffangbereich fallen und in dieser Weise aus dem Produktionsprozess entfernt werden.

Zudem besteht die Möglichkeit, die saugend gehaltene Leerdosenlage nach Einstellung des Grenzwertes senkrecht zu einer Transportrichtung über einen Aussonderungs- oder Auffangbereich zu führen, welcher Aussonderungs- oder Auffangbereich sich durch ein geringeres Höhenniveau auszeichnet, wobei fehlerhafte Leerdosen in den Aussonderungs- oder Auffangbereich fallen, und wobei die saugend gehaltenen mechanisch intakten und/oder unverformten Leerdosen anschließend in Transportrichtung der weiteren Verarbeitung zugeführt werden.

Denkbar wäre auch, dass man die angesaugte Leerdosenlage nur geringfügig anhebt und durch eine seitliche Verfahrbewegung die aus der Leerdosenlage gelösten fehlerhaften Leerdosen in einen seitlich stehenden Auffangbehälter abräumt.

Weiterhin kann eine Erkennung vorgesehen sein, mit welcher intakte Leerdosenlagen erkannt werden, d.h. Leerdosenlagen, welche nur mechanisch intakte und/oder unverformte Leerdosen umfassen. Beispielsweise erkennt eine Steuerungseinrichtung das Abfallen defekter Leerdosen aufgrund einer notwendigen Drucknachregulierung. Löst sich eine defekte Leerdose, dann muss der Saugdruck erhöht werden, um weiterhin den gewünschten Unterdruck für die gesamte Leerdosenlage zu erreichen. Enthält eine Leerdosenlage dagegen keine fehlerhaften Leerdosen, dann ist eine solche Nachregulierung nicht notwendig.

Die Steuerungseinrichtung kann dementsprechend ein schnelleres Überführen der Leergutlage zum Ablageplatz ansteuern, da kein Umweg über einen Aussonderungs- oder Auffangbereich bzw. keine zeitliche Verzögerung in einem Aussonderungs- oder Auffangbereich notwendig ist. Insbesondere kann vorgesehen sein, dass eine als intakt erkannte Leerdosenlage ohne Umweg über den Aussonderungs- oder Auffangbereich direkt dem Ablageplatz und/oder der weiteren Verarbeitung zugeführt wird, oder aber eine solche intakte Leerdosenlage wird ohne zeitliche Verzögerung zum Entfernen fehlerhafter Leerdosen dem Ablageplatz und/oder der weiteren Verarbeitung zugeführt.

Weiterhin wird eine Vorrichtung zum Handhaben von Leerdosenlagen beschrieben, welche einen Aufnahmeplatz, einen Ablageplatz, einen Lagengreifer, eine Einrichtung zur Aufnahme und/oder zum Entfernen von fehlerhaften Leerdosen und eine Steuerungseinrichtung umfasst.

Die Leerdosenlagen werden an dem Aufnahmeplatz bereitgestellt. Insbesondere werden mehrere auf einer Palette übereinandergestapelte Leerdosenlagen an einem Auflageplatz bereitgestellt.

Die intakten Leerdosen der Leerdosenlage werden anschließend zu einem Ablageplatz für die Leerdosen überführt, welcher Ablageplatz durch eine abführende Transporteinrichtung gebildet ist oder welchem Ablageplatz eine abführende Transporteinrichtung zugeordnet ist.

Die Leerdosenlage wird durch einen Lagengreifer erfasst, welcher dazu ausgestattet, eine komplette Leerdosenlage am Aufnahmeplatz saugend zu erfassen, zum Ablageplatz zu verbringen und an diesem freizugeben.

Die Steuerungseinrichtung dient insbesondere der Steuerung des Lagengreifers und gegebenenfalls weiterer Maschinenkomponenten. Hierzu umfasst die Steuerungseinrichtung ein Programm zur Steuerung des Lagengreifers, welches Programm zumindest die nachfolgenden Verfahrensparameter vorsieht. Zunächst erfolgt das Erfassen einer Leerdosenlage am Aufnahmeplatz durch Anlegen eines ersten Unterdrucks. Dieser Unterdruck wird gesteigert auf einen vordefinierten Grenzwert. Anschließend erfolgt die Freigabe von mechanisch intakten und/oder unverformten Leerdosen am Ablageplatz.

Die Einrichtung zur Aufnahme und/oder zum Entfernen von fehlerhaften Leerdosen kann einen Aussonderungs- oder Auffangbereich umfassen. Gemäß einer Ausführungsform ist dieser Aussonderungs- oder Auffangbereich im Bereich des Aufnahmeplatzes ausgebildet. Eine weitere Ausführungsform kann vorsehen, dass der Aussonderungs- oder Auffangbereich benachbart zum Aufnahmeplatz ausgebildet ist oder dass der Aussonderungs- oder Auffangbereich zwischen dem Aufnahmeplatz und dem Ablageplatz ausgebildet ist.

Beispielsweise kann der Aussonderungs- oder Auffangbereich auf einem Höhenniveau angeordnet sein, welcher sich unterhalb eines Höhenniveaus des Aufnahmeplatzes und/oder unterhalb eines Höhenniveaus des Ablageplatzes befindet.

Wenn die Leerdosenlagen übereinandergestapelt auf Paletten bereitgestellt werden, so ändert sich durch die Entstapelung die Höhe des Stapels. Entweder muss der Lagengreifer diesen Höhenunterschied entsprechend beim Erfassen der Leerdosenlage ausgleichen. Alternativ kann dem Aufnahmeplatz eine Einstelleinrichtung zur Einstellung eines Höhenniveaus des Aufnahmeplatzes zugeordnet sein, so dass sich die oberste, zu erfassende Leerdosenlage zum Erfassen immer in derselben Höhe befindet.

Eine Ausführungsform kann dementsprechend vorsehen, dass der Aufnahmeplatz, an welchem die Leerdosenlagen bereitgestellt werden, höhenverstellbar ausgebildet ist. Insbesondere ist vorgesehen, dass der Aufnahmeplatz in Abhängigkeit von der Beladung der Palette unterschiedliche Höhenniveaus einnehmen kann, damit die zu erfassende, oberste Leerdosen vorzugsweise immer in demselben Höhenniveau angeordnet ist.

Das Erkennen und Aussortieren der fehlerhaften Leerdosen kann im Zusammenhang der Entstapelung von übereinander angeordneter Leerdosenlagen erfolgen.

Eine weitere Ausführungsform kann vorsehen, dass die Leerdosenlagen auch erst entstapelt werden und anschließend zum Aussortieren der defekten Leerdosen entsprechend saugend erfasst und nach dem oben beschriebenen Verfahren gehandhabt werden.

Eine Ausführungsform sieht vor, dass eine Leerdosenlage angesaugt und saugend gehalten wird. Dabei kann der Unterdruck direkt auf den Grenzwert eingestellt werden. Nunmehr wird die saugend gehaltene Leerdosenlage angehoben. Die fehlerhaften Leerdosen verbleiben hierbei am Aufnahmeplatz und können durch geeignete Einrichtungen, beispielsweise ein Schiebeelement, aus dem Produktionsprozess entfernt werden. Die Leerdosenlage, welche nur noch intakte Leerdosen umfasst, wird nunmehr an den Ablageplatz überführt und an diesem abgesetzt.

Weiterhin können bei einer Ausführungsform der Erfindung Aufnahmeplatz und Ablageplatz zusammenfallen, beispielsweise wird eine angesaugte Lage angehoben, die rausgefallenen Leerdosen werden über einen Schieber oder ähnliches aus dem Bereich rausgeschoben und die Lage wird wieder abgesetzt.

Die Erfindung verwendet zum Abheben der Leerdosenlage gemäß einer Ausführungsform einen verfahrgesteuerten Saugplattengreifer, welcher mit einer Saugfläche für die Leerdosen der Leerdosenlage ausgestattet ist. Insbesondere weist der Lagengreifer an seiner Unterseite eine flächig angeordnete, mit Sauglöchern ausgestattete elastische Schaumplatte auf, beispielsweise einen elastischen Saugschaum oder eine mit Sauglöchern ausgestattetet Gummiplatte oder dergleichen. Eine Aufnahmeverbindung des Lagengreifers an die Leerdosen wird derart ausgeführt, dass der Saugschaum von oben auf den Rand der oberseitigen Öffnungen der Leerdosen aufdrückt. Dadurch wird eine luftdichte bündige Andrückverbindung zwischen dem Saugschaum und den Leerdosen ausgebildet.

Bei fehlerhaften Leerdosen, insbesondere bei stark verknickten Leerdosen o.ä., kann es vorkommen, dass der Rand der oberseitigen Öffnung nicht in einer Horizontalebene angeordnet ist. In diesem Fall kann es vorkommen, dass eine derart beschädigte Leerdose gar nicht durch den Lagengreifer erfasst werden kann und somit am Aufnahmeplatz verbleibt.

Bei fehlerhaften Leerdosen, insbesondere bei einer Verknickung oder anderen Beschädigung in der ansonsten kontinuierlichen Außenform der fehlerhaften Leerdose oder auch beim Vorhandensein feiner Risse, wirken bei der Erhöhung des Unterdrucks Zugkräfte im Inneren der fehlerhaften Leerdosen, welche bewirken, dass sich die fehlerhaften Leerdosen durch mechanisches Zusammenziehen plastisch verformen. Dabei verformt sich der Bereich der oberseitigen Öffnung der fehlerhaften Leerdose, so dass diese nicht mehr an der Saugfläche abschließt. Damit öffnet sich das geschlossene Systems und die fehlerhafte Leerdose fällt von der Saugfläche ab, während die intakten Leerdosen weiterhin saugend an der Saugfläche gehalten werden.

In Abhängigkeit vom jeweiligen Beschädigungsgrad der fehlerhaften Leerdose wird sich diese ab einem gewissen Unterdruckwert zu verformen beginnen. In einer maximalen Ausprägung der Beschädigung kann die defekte Leerdose auch komplett in sich zusammenfallen bzw. implodieren.

Dagegen kann eine intakte Leerdose mit einem deutlich höheren Unterdruck, d.h. einer deutlich größeren Druckdifferenz im Vergleich zum Normaldruck, beschickt werden. Um eine Beschädigung von intakten Leerdosen zu vermeiden, ist hierbei darauf zu achten, dass der für die jeweiligen Leerdosen charakteristische physikalische Knickpunkt nicht erreicht wird.

Die Saugfläche kann auch durch eine an die jeweilig zu erfassende Leerdosenlage angepasste mit Sauglöchern ausgestattete elastische Schaumplatte gebildet sein, welche mit Unterdruck versorgt werden kann. Die Schaumplatte wird von oben auf die oberseitigen Öffnungen der Leerdosen angedrückt und der Unterdruck angelegt.

Alternativ kann der Lagengreifer auch eine flächige Sauggummiplatte oder eine Mehrzahl von flächig und in entsprechender Anzahl und Anordnung angeordneten Saugnäpfen umfassen.

Wenn die Saugfläche auf den oberseitigen Öffnungen der Leerdosen aufliegt oder wenn die Saugnäpfe auf den oberseitigen Öffnungen der Leerdosen aufliegen und die Leerdosen der Leerdosenlage entsprechend angesaugt werden, kann das Unterdruckniveau bis zu einem vordefinierten Grenzwert gesteigert werden.

Dabei verformen sich die fehlerhaften Leerdosen und/oder implodieren, wodurch die zur Ansaugung notwendige Anlage-Dichtigkeit im Bereich der oberseitigen Öffnung verloren geht. Die fehlerhaften Leerdosen werden dementsprechend nicht mehr saugend gehalten, sondern fallen aufgrund der Schwerkraft nach unten aus der Leerdosenlage hinaus.

Zur Stabilitätserhaltung des verbleibenden Stapels an Leerdosenlagen und insbesondere in unteren Leerdosenlagen und/oder zur Positionserhaltung einer zwischen der übereinandergestapelten Leerdosenlagen zum Schutz und/oder zur Stabilisierung angeordneten Zwischenlage kann eine stapelumlaufende Zentrierung vorteilhaft sein.

Weiterhin kann am Aufnahmeplatz ein zusätzlicher, insbesondere freistehender, Zwischenlagenausheber vorgesehen sein, welcher insbesondere eine stehende Schiebeplatte am Zwischenlagen-Greiferkopf umfasst und dazu vorgesehen ist, nach dem seitlichen Entfernen von fehlerhaften Leerdosen in einen Auffangbehälter die jeweilige Zwischenlage abzuheben.

Im Folgenden sollen Ausführungsbeispiele die Erfindung und ihre Vorteile anhand der beigefügten Figuren näher erläutern. Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind.
Figuren 1 bis 3 zeigen eine erste Ausführungsform einer Vorrichtung und eines Verfahrens zum Handhaben von Leerdosenlagen.
Fig. 4 zeigt eine zweite Ausführungsform einer Vorrichtung und eines Verfahrens zum Handhaben von Leerdosenlagen.
Figuren 5A bis 5E zeigen eine dritte Ausführungsform einer Vorrichtung und eines Verfahrens zum Handhaben von Leerdosenlagen.
Fig. 6 zeigt eine vierte Ausführungsform einer Vorrichtung zum Handhaben von Leerdosenlagen.

Für gleiche oder gleich wirkende Elemente der Erfindung werden identische Bezugszeichen verwendet. Ferner werden der Übersicht halber nur Bezugszeichen in den einzelnen Figuren dargestellt, die für die Beschreibung der jeweiligen Figur erforderlich sind. Die dargestellten Ausführungsformen stellen lediglich Beispiele dar, wie die erfindungsgemäße Vorrichtung oder das erfindungsgemäße Verfahren ausgestaltet sein können und stellen keine abschließende Begrenzung dar.

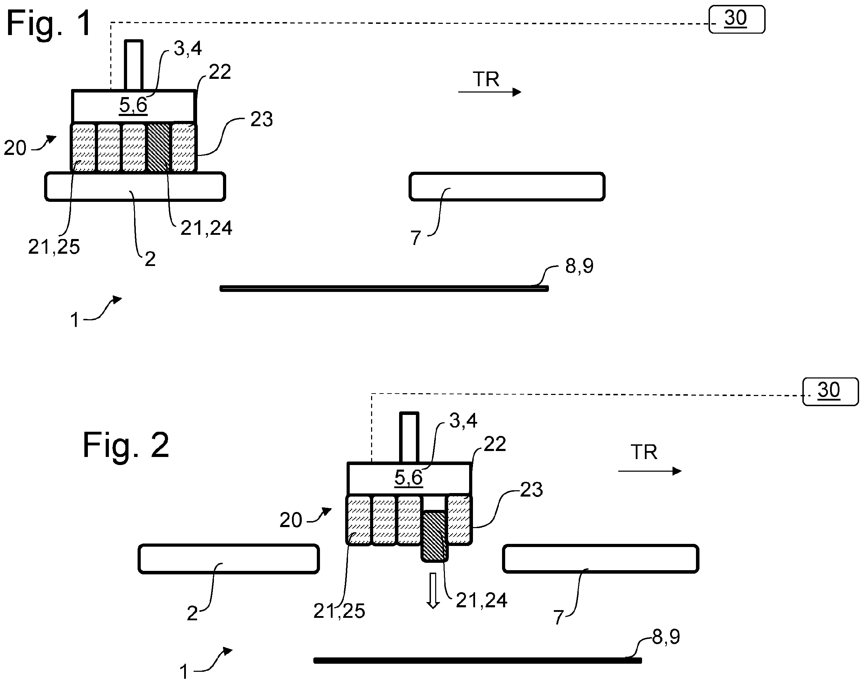

Die Figuren 1 bis 3 zeigen eine erste Ausführungsform eines Verfahrens zum Handhaben von Leerdosenlagen 20 anhand einer schematisch dargestellten Vorrichtung 1 zum Handhaben von Leerdosenlagen 20.

An einem Aufnahmeplatz 2 werden Leerdosenlagen 20 bereitgestellt. Im vorliegenden Beispiel wird eine einzelne Leerdosenlage 20 bereitgestellt. In der Regel werden mehrere auf einer Palette übereinandergestapelte Leerdosenlagen 20 an dem Aufnahmeplatz 2 bereitgestellt.

Die Leerdosenlage 20 umfasst eine Mehrzahl an regelmäßig angeordneten Leerdosen 21 mit oberseitigen Öffnungen 22 und seitlichen Mantelflächen 23.

Es kann vorkommen, dass die Leerdosenlage 20 defekte oder fehlerhafte Leerdosen 24 umfasst. Unter defekten oder fehlerhaften Leerdosen 24 sind insbesondere solche zu verstehen, welche eingeknickt und/oder eingedellt sind oder sonstige Verformungen aufweisen. Auch stellen Risse im Leerdosenmaterial einen Fehler dar, der zu fehlerhaften Produkten führen würde. Somit ist es auch notwendig, solche fehlerhaften Leerdosen 24 zu identifizieren und vor der weiteren Verarbeitung aus dem Produktionsprozess auszuschleusen.

Zum Erfassen der Leerdosenlage 20 werden vorzugsweise alle Leerdosen 21 einer Leerdosenlage 20, d.h. sowohl intakte Leerdosen 25 als auch fehlerhafte Leerdosen 24, durch einen Lagengreifer 3 erfasst. Der Lagengreifer 3 ist insbesondere ausgestattet, die Leerdosenlage 20 am Aufnahmeplatz 2 saugend zu erfassen, zum Ablageplatz 7 zu verbringen und an diesem freizugeben.

Insbesondere werden die Leerdosen 21 durch Anlegen eines Unterdrucks an den oberseitigen Öffnungen 22 angesaugt und saugend gehalten.

Vorzugsweise ist der Lagengreifer 3 als verfahrgesteuerter Saugplattengreifer 4 ausgebildet, welcher mit einer Saugfläche 5 für Leerdosenlage 20 ausgestattet ist. Insbesondere weist der Lagengreifer 3 an seiner Unterseite einen flächig angeordneten elastischen Saugschaum 6 auf. Eine Aufnahmeverbindung des Lagengreifers 3 an die Leerdosen 21 wird derart ausgeführt, dass der Saugschaum 6 von oben auf den Rand der oberseitigen Öffnungen 22 aufdrückt. Dadurch wird eine luftdichte bündige Andrückverbindung zwischen der Saugfläche 4 und den Leerdosen 21 ausgebildet.

Die Saugfläche 5 kann auch durch eine an die jeweilig zu erfassende Leerdosenlage 20 angepasste mit Sauglöchern ausgestattete elastische Schaumplatte gebildet sein, an welche mit Unterdruck versorgt werden kann. Die Schaumplatte wird von oben auf die oberseitigen Öffnungen der Leerdosen angedrückt und der Unterdruck angelegt.

Alternativ kann der Lagengreifer 3 auch eine flächige Sauggummiplatte oder eine Mehrzahl von flächig und in entsprechender Anzahl und Anordnung angeordnete Saugnäpfe umfassen.

Der Lagengreifer 3 überführt nunmehr die angesaugte Leerdosenlage 20 zu einem Ablageplatz 7, wobei der Lagengreifer 3 die angesaugte Leerdosenlage 20 über einen Aussonderungs- oder Auffangbereich 8 führt.

Wenn sich der Lagengreifer 3 mit der angesaugten Leerdosenlage 20 oberhalb des Aussonderungs- oder Auffangbereichs 8 befindet, dann wird der saugende Unterdruck wird bis zu vordefinierten einem Grenzwert gesteigert.

Wenn im vorliegenden Zusammenhang von einer Erhöhung des Unterdrucks gesprochen wird, so ist darunter zu verstehen, dass die Druckdifferenz zwischen dem Unterdruck und dem Normaldruck weiter erhöht wird.

Der Grenzwert ist abhängig von den jeweiligen Leerdosen 21 und bestimmt sich beispielsweise aufgrund von deren Raumvolumen, dem Leerdosenmaterial, der Wandstärke und gegebenenfalls weiterer Parameter.

Der Grenzwert wird durch einen Unterdruck gebildet, bei welchem die auf die Mantelflächen 23 der Leerdosen 21 wirkenden Druckkräfte von intakten Leerdosen 25 durch die Eigenstabilität zumindest soweit kompensiert werden, dass sich die Mantelflächen 23 der intakten Leerdosen 25 nach Aufheben oder Reduzieren des Unterdrucks elastisch in ihre Ausgangskontur zurückverformen.

Insbesondere werden mechanisch intakte und/oder unverformte Leerdosen 25 bis zum Grenzwert des Unterdrucks weiterhin saugend gehalten.

Dagegen werden fehlerhafte Leerdosen 24 vor oder bei Erreichen des Grenzwertes plastisch verformt. Insbesondere können fehlerhafte Leerdosen 24 vor oder bei Erreichen des Grenzwertes implodieren. Dies wird durch Zugkräfte im Inneren der fehlerhaften Leerdosen 24 bewirkt, welche Zugkräfte bei der Erhöhung des Unterdrucks bewirken, dass sich die fehlerhaften Leerdosen 24 durch mechanisches Zusammenziehen plastisch verformen. Dabei verformt sich in der Regel der Bereich der oberseitigen Öffnung 22 der fehlerhaften Leerdose 24 derart, dass diese nicht mehr an der Saugfläche 5 abschließt. Damit öffnet sich das geschlossene Systems und die fehlerhafte Leerdose 24 fällt von der Saugfläche 5 ab, während die intakten Leerdosen 25 weiterhin saugend gehalten werden.

In Abhängigkeit vom jeweiligen Beschädigungsgrad der fehlerhaften Leerdose 24 wird sich diese ab einem gewissen Unterdruckwerte zu verformen beginnen. Dagegen kann eine intakte Leerdose 25 mit einem deutlich höheren Unterdruck, d.h. einer deutlich größeren Druckdifferenz im Vergleich zum Normaldruck, beschickt werden. Um eine Beschädigung von intakten Leerdosen 25 zu vermeiden, ist hierbei darauf zu achten, dass der für die jeweiligen Leerdosen 21 charakteristische physikalische Knickpunkt nicht erreicht wird.

Aufgrund der Verformung oder Implosion der fehlerhaften Leerdosen 24 können diese durch den Lagengreifer 3 nicht mehr ausreichend saugend gehalten werden. Die fehlerhaften Leerdosen 24 lösen sich aus der Leerdosenlage 20 und insbesondere aus der saugenden Verbindung mit dem Lagengreifer 3 und fallen aufgrund der Schwerkraft nach unten in den Aussonderungs- oder Auffangbereich 8 (Fig. 2). Der Aussonderungs- oder Auffangbereich 8 umfasst beispielsweise einen Fördereinrichtung 9, welche die aussortierten defekten Leerdosen 24 in einer Transportrichtung TR aus dem Aussonderungs- oder Auffangbereich 8 entfernt.

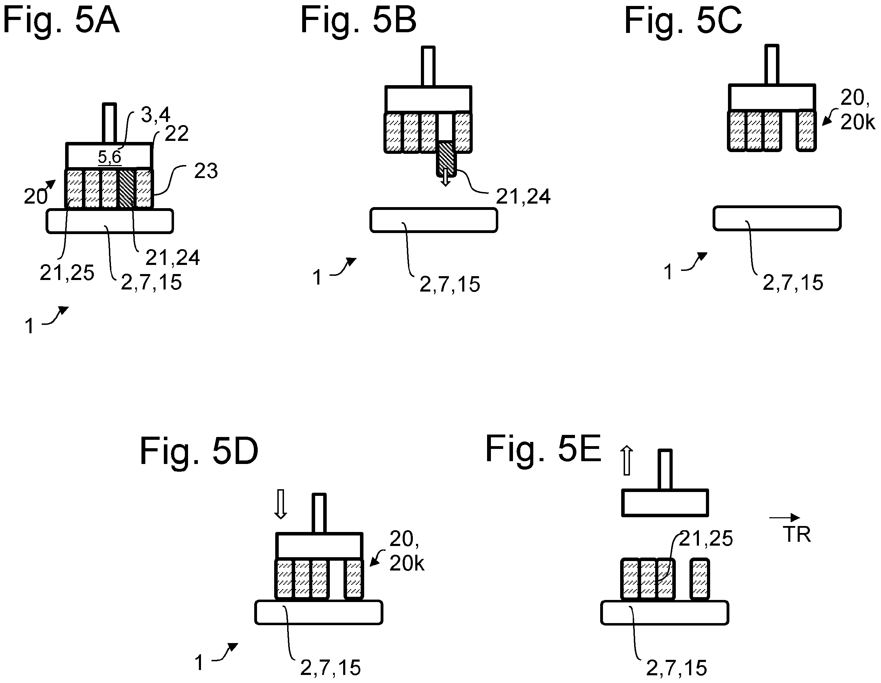

Der Aussonderungs- oder Auffangbereich 8 kann auch durch einen Auffangbehälter 13 gebildet werden, in welchem die fehlerhaften Leerdosen 24 gesammelt werden (vergleiche Fig. 5).

Da der Aussonderungs- oder Auffangbereich 8 sich durch ein geringeres Höhenniveau auszeichnet als der Aufnahmeplatz 2, muss die Leerdosenlage 20 beim Erfassen am Aufnahmeplatz 2 und beim Überführen zum Ablageplatz 7 nicht wesentlich angehoben werden. Eine im wesentliche horizontale Verschiebebewegung in einer Transportrichtung TR ist ausreichend, wobei ein geringfügiges Anheben der erfassten Leerdosenlage 20 zum Überwinden der Reibung vorteilhaft sein kann.

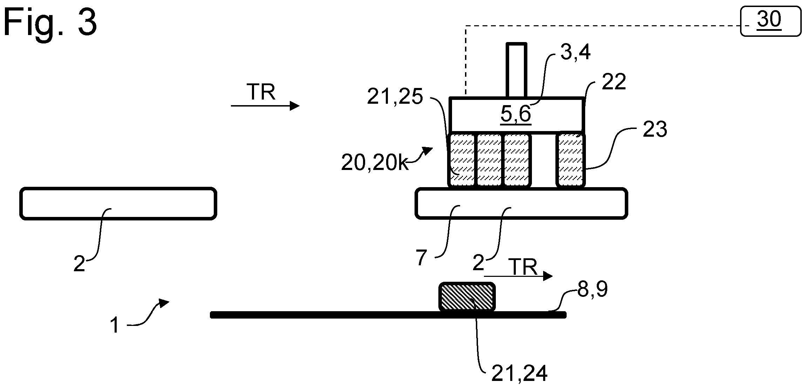

Die derart korrigierte Leerdosenlage 20k, welche nur noch intakte Leerdosen 25 umfasst, wird nunmehr vom Lagengreifer 3 am Ablageplatz 3 abgesetzt und freigegeben (Fig. 3). Der Ablageplatz 7 kann beispielsweise durch eine abführende Transporteinrichtung gebildet werden oder dem Ablageplatz 7 kann eine abführende Transporteinrichtung 7 zugeordnet sein.

Die intakten Leerdosen 25 können nunmehr der weiteren Verarbeitung zugeführt werden (nicht dargestellt).

In den Figuren 1 bis 3 ist weiterhin eine Steuerungseinrichtung 30 dargestellt, welche den Lagengreifer 3 und gegebenenfalls weitere Maschinenkomponenten ansteuert. Insbesondere umfasst die Steuerungseinrichtung 30 ein Programm zur Steuerung des Lagengreifer 3, welches Programm zumindest folgende Verfahrensparameter vorsieht:
- Erfassen einer Leerdosenlage 20 am Aufnahmeplatz 2 durch Anlegen eines ersten Unterdrucks;
- Steigern des Unterdrucks auf einen vordefinierten Grenzwert;
- Freigabe einer korrigierten Leerdosenlage 20k mit mechanisch intakten und/oder unverformten Leerdosen 25 am Ablageplatz 7.

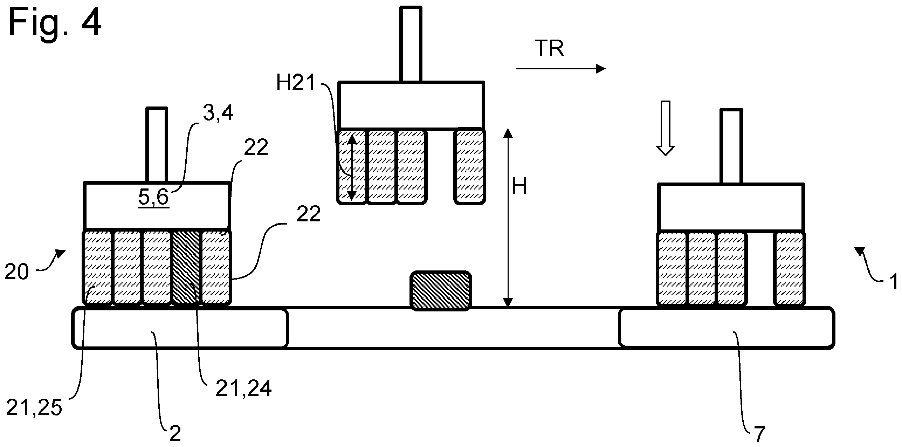

Die Fig. 4 zeigt eine zweite Ausführungsform einer Vorrichtung 1 und eines Verfahrens zum Handhaben von Leerdosenlagen 20. Hierbei wird die erfasste Leerdosenlage 20 in Transportrichtung TR vom Aufnahmeplatz 2 zum Ablageplatz 7 bewegt, wobei die erfasste Leerdosenlage 20 auf eine Höhe H angehoben wird, welche Höhe H mindestens einer halben Dosenhöhe H21 entspricht.

Während des Transports in Transportrichtung TR wird der Unterdruck auf den Grenzwert erhöht, so dass sich defekte Leerdosen 24 aus der Leerdosenlage 20 lösen.

Die defekten aus der Leerdosenlage 20 herausgelösten Leerdosen 24 werden beispielsweise über einen seitlich wirkenden Pusher (nicht dargestellt) o.ä. entfernt und in einen Auffangbehälter o.ä. überführt. Die korrigierte Leerdosenlage 20k wird durch Absenken im Ablageplatz 7 angeordnet und vom Lagengreifer 3 für die weitere Verarbeitung freigegeben,

Die Figuren 5A bis 5E zeigen eine dritte Ausführungsform einer Vorrichtung 1 und eines Verfahrens zum Handhaben von Leerdosenlagen 20. Hierbei sind Aufnahmeplatz 2 und Ablageplatz 7 gleich und bilden einen kombinierten Aufnahmeplatz/Ablageplatz 15. Diese Ausführungsform ist insbesondere dann vorteilhaft, wenn die Leerdosenlagen 20 bereits entstapelt in Einzellagen vorliegen und somit eine Entstapelung nicht notwendig ist. Ansonsten kann es vorteilhaft sein, das Aussondern fehlerhafter Leerdosen direkt in den Entstapelungsvorgang zu integrieren.

Gemäß der in den Figuren 5 dargestellten Ausführungsform wird die Leerdosenlage 20 durch den Lagengreifer 3 angesaugt und saugend gehalten (Fig. 5A). Anschließend ist vorgesehen, dass die Leerdosenlage 20 angehoben wird, so dass fehlerhafte Leerdosen 24, welche sich bei einer Erhöhung des Unterdrucks aus der Leerdosenlage 20 lösen, herunterfallen (Fig. 5B). Die heruntergefallenen fehlerhaften Leerdosen 24 werden durch einen Pusher o.ä. (nicht dargestellt) vom kombinierten Aufnahmeplatz/Ablageplatz 15 entfernt (Fig. 5C) und die korrigierte Leerdosenlage 20 wird nunmehr im kombinierten Aufnahmeplatz/Ablageplatz 15 wieder abgesetzt (Fig. 5D). Die intakten Leerdosen 25 werden nach Freigabe durch den Lagengreifer 3 in Transportrichtung TR abgeführt und der weiteren Verarbeitung zugeführt (Fig. 5E).

Bei der hier dargestellten Ausführungsform kann der Unterdruck direkt beim Ansaugen der Leerdosenlage 20 auf den Grenzwert eingestellt werden. Nunmehr wird die saugend gehaltene Leerdosenlage 20 angehoben. Die fehlerhaften Leerdosen 24 verbleiben hierbei direkt am kombinierten Aufnahme-/Ablageplatz 15 und können durch geeignete Einrichtungen, beispielsweise ein Schiebeelement, aus dem Produktionsprozess entfernt werden.

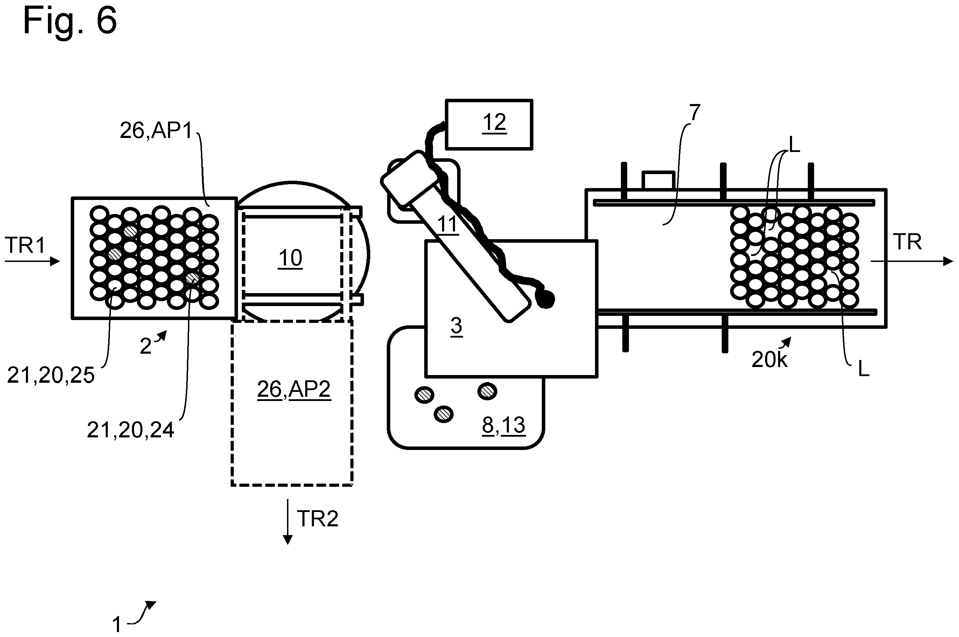

Die schematische Draufsicht der Fig. 6 zeigt eine vierte Ausführungsform einer Vorrichtung 1 zum Handhaben von Leerdosenlagen 20. Die Leerdosenlagen 20 werden hierbei insbesondere übereinandergestapelt auf Paletten 26 bereitgestellt. Die mit Leerdosenlagen 20 beladenen Paletten 20 werden in einer ersten Transportrichtung TR1 zugeführt. Nach dem vollständigen Entladen wird die Leerpalette 26 in einer zweiten Transportrichtung TR2 abgeführt. Insbesondere ist die Palette 26 auf einem Palettendrehsegment 10 angeordnet, durch welches die abgeräumte Palette 26 von einer den Aufnahmeplatz 2 bildenden ersten Arbeitsposition AP1 in eine zweite Arbeitsposition AP2 überführt werden kann, aus welcher die leere Palette 26 abtransportiert werden kann.

Der Lagengreifer 3 ist insbesondere an einem drehbeweglichen Roboterarm 11 angeordnet, welcher den Lagengreifer 3 zwischen dem Aufnahmeplatz 2 und dem Ablageplatz 7 bewegen kann.

Zur Erzeugung des zum Ansaugen der Leerdosenlage 20 und zum Aussondern defekter Leerdosen 24 notwendige Unterdruck, ist dem Lagengreifer 3 ein Seitenkanalverdichter 12 zugeordnet.

Zwischen dem Aufnahmeplatz 2 dem Ablageplatz 7 ist ein Aussonderungs- oder Auffangbereich 8 mit einem Auffangbehälter 13 vorgesehen. Der Lagengreifer 3 positioniert die angesaugte Leerdosenlage 20 oberhalb des Aussonderungs- oder Auffangbereichs 8 und der Unterdruck wird auf den Grenzwert gesteigert, so dass sich die fehlerhaften Leerdosen 24 aus der Leerdosenlage 20 lösen und in den Auffangbehälter 13 fallen.

Die korrigierte Leerdosenlage 20k kann nunmehr in Transportrichtung TR aus dem Ablageplatz 7 abgeführt und der weiteren Verarbeitung zugeführt werden. Die durch die aussortierten fehlerhaften Leerdosen 24 innerhalb der korrigierten Leerdosenlage 20k ausgebildeten Lücken sind mit einem L gekennzeichnet.

Wenn die Leerdosenlagen 20 übereinandergestapelt auf Paletten 26 bereitgestellt werden, so ändert sich durch die Entstapelung die Höhe des Stapels. Entweder muss der Lagengreifer 3 diesen Höhenunterschied entsprechend beim Erfassen der Leerdosenlage 20 ausgleichen. Alternativ kann dem Aufnahmeplatz 2 eine Einstelleinrichtung (nicht dargestellt) zur Einstellung eines Höhenniveaus des Aufnahmeplatzes 2 zugeordnet sein, so dass sich die oberste, zu erfassende Leerdosenlage 20 zum Erfassen immer in derselben Höhe befindet.

Bei den vorbeschriebenen Verfahren und Vorrichtungen 1 kann weiterhin eine Erkennung vorgesehen sein, mit welcher intakte Leerdosenlagen 20 erkannt werden, d.h. Leerdosenlagen 20, welche nur mechanisch intakte und/oder unverformte Leerdosen 25 umfassen. Beispielsweise erkennt die im Zusammenhang mit den Figuren 1 bis 3 beispielhaft dargestellte Steuerungseinrichtung 30 das Abfallen defekter Leerdosen 24 aufgrund einer notwendigen Drucknachregulierung. Löst sich eine defekte Leerdose 24, dann muss der Saugdruck erhöht werden, um weiterhin den gewünschten Unterdruck für die gesamte Leerdosenlage 20 zu erreichen. Enthält eine Leerdosenlage 20 dagegen keine fehlerhaften Leerdosen 24, dann ist eine solche Nachregulierung nicht notwendig.

Die Steuerungseinrichtung 30 kann dementsprechend ein schnelleres Überführen der Leergutlage 20 zum Ablageplatz 7 ansteuern, da kein Umweg über bzw. keine zeitliche Verzögerung in einem Aussonderungs- oder Auffangbereich 8 notwendig ist. Insbesondere wird eine solche intakte Leerdosenlage 20 ohne Umweg über den Aussonderungs- oder Auffangbereich 8 direkt dem Ablageplatz 7 und/oder der weiteren Verarbeitung zugeführt wird, oder aber eine solche intakte Leerdosenlage 20 wird ohne zeitliche Verzögerung zum Entfernen fehlerhafter Leerdosen 24 dem Ablageplatz 7 und/oder der weiteren Verarbeitung zugeführt.

Zur Stabilitätserhaltung des verbleibenden Stapels an Leerdosenlagen 20 und insbesondere unterer auf der Palette 26 angeordneter Leerdosenlagen 20 und/oder zur Positionserhaltung einer zwischen der übereinandergestapelten Leerdosenlagen 20 zum Schutz und/oder zur Stabilisierung angeordneten Zwischenlage kann eine stapelumlaufende Zentrierung vorteilhaft sein (nicht dargestellt).

Weiterhin kann am Aufnahmeplatz 2 ein zusätzlicher, insbesondere freistehender Zwischenlagenausheber vorgesehen sein, welcher insbesondere eine stehende Schiebeplatte am Zwischenlagen-Greiferkopf umfasst und dazu vorgesehen ist, nach dem seitlichen Entfernen von fehlerhaften Leerdosen 24 in einen Auffangbehälter 13 die jeweilige Zwischenlage abzuheben.

Die Ausführungsformen, Beispiele und Varianten der vorhergehenden Absätze, die Ansprüche oder die folgende Beschreibung und die Figuren, einschließlich ihrer verschiedenen Ansichten oder jeweiligen individuellen Merkmale, können unabhängig voneinander oder in beliebiger Kombination verwendet werden. Merkmale, die in Verbindung mit einer Ausführungsform beschrieben werden, sind für alle Ausführungsformen anwendbar, sofern die Merkmale nicht unvereinbar sind.

Wenn auch im Zusammenhang der voranstehenden Figurenbeschreibung generell von "schematischen" Darstellungen und Ansichten die Rede ist, so ist damit keineswegs gemeint, dass die Figurendarstellungen und deren Beschreibung hinsichtlich der Offenbarung der Erfindung von untergeordneter Bedeutung sein sollen. Der Fachmann ist durchaus in der Lage, aus den schematisch und abstrakt gezeichneten Darstellungen genug an Informationen zu entnehmen, die ihm das Verständnis der Erfindung erleichtern, ohne dass er etwa aus den gezeichneten und möglicherweise nicht exakt maßstabsgerechten Größenverhältnissen der Leerdosen, Leerdosenlagen und/oder Teilen der Vorrichtung oder anderer gezeichneter Elemente in irgendeiner Weise in seinem Verständnis beeinträchtigt wäre. Die Figuren ermöglichen es dem Fachmann als Leser somit, anhand der konkreter erläuterten Umsetzungen des erfindungsgemäßen Verfahrens und der konkreter erläuterten Funktionsweise der erfindungsgemäßen Vorrichtung ein besseres Verständnis für den in den Ansprüchen sowie im allgemeinen Teil der Beschreibung allgemeiner und/oder abstrakter formulierten Erfindungsgedanken abzuleiten.

Die Erfindung wurde unter Bezugnahme auf eine bevorzugte Ausführungsform beschrieben. Es ist jedoch für einen Fachmann vorstellbar, dass Abwandlungen oder Änderungen der Erfindung gemacht werden können, ohne dabei den Schutzbereich der nachstehenden Ansprüche zu verlassen.

### Bezugszeichenliste

- 1: Vorrichtung
- 2: Aufnahmeplatz
- 3: Lagengreifer
- 4: Saugplattengreifer
- 5: Saugfläche
- 6: Saugschaum
- 7: Ablageplatz
- 8: Aussonderungs- oder Auffangbereich
- 9: Fördereinrichtung
- 10: Palettendrehsegment
- 11: Roboterarm
- 12: Seitenkanalverdichter
- 13: Auffangbehälter
- 15: kombinierter Aufnahmeplatz/Ablageplatzv
- 20: Leerdosenlage
- 20k: korrigierte Leerdosenlage
- 21: Leerdose
- 22: oberseitige Öffnung
- 23: seitliche Mantelfläche
- 24: fehlerhafte / defekte Leerdose
- 25: intakte Leerdose
- 26: Palette
- 30: Steuerungseinrichtung

- H: Höhe
- H21: Dosenhöhe
- L: Lücke
- TR: Transportrichtung
- TR1: erste Transportrichtung
- TR2: zweite Transportrichtung

## Patentansprüche

1. Verfahren zum Handhaben von Leerdosenlagen (20), welche Leerdosenlagen (20) eine Mehrzahl an regelmäßig angeordneten Leerdosen (21) mit oberseitigen Öffnungen (22) und seitlichen Mantelflächen (23) aufweisen,
welche Leerdosenlagen (20) an einem Aufnahmeplatz (2) bereitgestellt werden, bei welchem Verfahren alle Leerdosen (21) einer Leerdosenlage (20) durch Anlegen von Unterdruck an den oberseitigen Öffnungen (22) der Leerdosen (21) angesaugt und saugend gehalten werden,
wobei der saugende Unterdruck bis zu einem Grenzwert gesteigert wird,
**dadurch gekennzeichnet, dass** bei dem Grenzwert die auf die Mantelflächen (23) der Leerdosen (21) wirkenden Druckkräfte von intakten Leerdosen (21) durch deren Eigenstabilität zumindest soweit kompensiert werden, dass sich die Mantelflächen (23) nach Aufheben oder Reduzieren des Unterdrucks elastisch in ihre Ausgangskontur zurückverformen.

2. Verfahren nach Anspruch 1, wobei mechanisch intakte und/oder unverformte Leerdosen (21) bis zum Grenzwert des Unterdrucks weiterhin saugend gehalten werden, während fehlerhafte Leerdosen (21) vor oder bei Erreichen des Grenzwertes plastisch verformt werden.

3. Verfahren nach Anspruch 1, wobei mechanisch intakte und/oder unverformte Leerdosen (21) bis zum Grenzwert des Unterdrucks weiterhin saugend gehalten werden, während fehlerhafte Leerdosen (21) vor oder bei Erreichen des Grenzwertes implodieren und sich dabei aus der Leerdosenlage (20) lösen.

4. Verfahren nach einem der voranstehenden Ansprüche, wobei die saugend gehaltenen mechanisch intakten und/oder unverformten Leerdosen (21) einem Ablageplatz (7) und/oder einer weiteren Verarbeitung zugeführt werden.

5. Verfahren nach einem der voranstehenden Ansprüche, wobei die Leerdosenlage (20) mit einem ersten Saugdruck angesaugt und saugend gehalten wird, wobei die angesaugte Leerdosenlage (20) oberhalb eines Auffangbehälters (13) angeordnet wird, wonach der saugende Unterdruck auf den Grenzwert gesteigert wird, wobei sich fehlerhafte Leerdosen (21) aus der Leerdosenlage (20) lösen und in den Auffangbehälter (13) fallen, wobei die weiterhin saugend gehaltenen, mechanisch intakten und/oder unverformten Leerdosen (21) der weiteren Verarbeitung zugeführt werden.

6. Verfahren nach einem der voranstehenden Ansprüche, wobei die saugend gehaltenen Leerdosenlage (20) angehoben werden, um die fehlerhaften Leerdosen (21) für die Entfernung aus dem Produktionsprozess zu isolieren oder wobei die saugend gehaltenen Leerdosenlage (20) nach Einstellung des Grenzwertes über einen Aussonderungs- oder Auffangbereich (8) geführt wird, welcher Aussonderungs- oder Auffangbereich (8) sich durch ein geringeres Höhenniveau auszeichnet, wobei fehlerhafte Leerdosen (21) in den Aussonderungs- oder Auffangbereich (8) fallen und aus dem Produktionsprozess entfernt werden.

7. Verfahren nach einem der Ansprüche 1 bis 5, wobei die saugend gehaltene Leerdosenlage (20) nach Einstellung des Grenzwertes senkrecht zu einer Transportrichtung (TR) über einen Aussonderungs- oder Auffangbereich (8) geführt werden, welcher Aussonderungs- oder Auffangbereich (8) sich durch ein geringeres Höhenniveau auszeichnet, wobei fehlerhafte Leerdosen (21) in den Aussonderungs- oder Auffangbereich (8) fallen, und wobei die saugend gehaltenen mechanisch intakten und/oder unverformten Leerdosen (21) anschließend in Transportrichtung (TR) der weiteren Verarbeitung zugeführt werden.

8. Verfahren nach einem der Ansprüche 6 oder 7, bei welchem erkannt wird, wenn eine saugend gehaltene Leerdosenlage (20) nur mechanisch intakte und/oder unverformte Leerdosen (21) umfasst,
wobei eine solche Leerdosenlage (20) ohne Umweg über den Aussonderungs- oder Auffangbereich (8) direkt dem Ablageplatz (7) und/oder der weiteren Verarbeitung zugeführt wird, oder
wobei eine solche Leerdosenlage (20) ohne zeitliche Verzögerung zum Entfernen fehlerhafter Leerdosen (21) dem Ablageplatz (7) und/oder der weiteren Verarbeitung zugeführt wird.

9. Vorrichtung (1) zum Handhaben von Leerdosenlagen (20), welche Leerdosenlagen (20) eine Mehrzahl an regelmäßig angeordneten Leerdosen (21) mit oberseitigen Öffnungen (22) und seitlichen Mantelflächen (23) aufweisen, die Vorrichtung (1) umfassend
einen Aufnahmeplatz (2), an welchem die Leerdosenlagen (20) bereitgestellt werden,
einem Ablageplatz (7) für die Leerdosen (21), welcher Ablageplatz (7) durch eine abführende Transporteinrichtung gebildet ist oder welchem Ablageplatz (7) eine abführende Transporteinrichtung zugeordnet ist,
einen Lagengreifer (3), welcher Lagengreifer (3) ausgestattet ist, die Leerdosenlage (20) am Aufnahmeplatz (2) saugend zu erfassen, und welcher Lagengreifer (3) ausgestattet, die Leerdosenlage (20) zum Ablageplatz (7) zu verbringen und dort freizugeben,
eine Steuerungseinrichtung (30) zur Steuerung des Lagengreifers (3)und weiterer Maschinenkomponenten,
**dadurch gekennzeichnet, dass** die Vorrichtung (1) eine Einrichtung zur Aufnahme und/oder zum Entfernen von fehlerhaften Leerdosen umfasst,
wobei die Steuerungseinrichtung (30) ein Programm zur Steuerung des Lagengreifers (3) umfasst, welches Programm folgende Verfahrensparameter vorsieht:
- Erfassen einer Leerdosenlage (20) am Aufnahmeplatz (2) durch Anlegen eines ersten Unterdrucks;
- Steigern des Unterdrucks auf einen vordefinierten Grenzwert,
bei welchem Grenzwert die auf die Mantelflächen (23) der Leerdosen (21) wirkenden Druckkräfte von intakten Leerdosen (21) durch deren Eigenstabilität zumindest soweit kompensiert werden, dass sich die Mantelflächen (23) nach Aufheben oder Reduzieren des Unterdrucks elastisch in ihre Ausgangskontur zurückverformen;
- Freigabe von mechanisch intakten und/oder unverformten Leerdosen (21) am Ablageplatz (7).

10. Vorrichtung (1) nach Anspruch 9, wobei die Einrichtung zur Aufnahme und/oder zum Entfernen von fehlerhaften Leerdosen (21) einen Aussonderungs- oder Auffangbereich (8) umfasst, welcher Aussonderungs- oder Auffangbereich (8) im Bereich des Aufnahmeplatzes (2) ausgebildet ist, oder
welcher Aussonderungs- oder Auffangbereich (8) benachbart zum Aufnahmeplatz (2) ausgebildet ist, oder
welcher Aussonderungs- oder Auffangbereich (8) zwischen dem Aufnahmeplatz (2) und dem Ablageplatz (7) ausgebildet ist.

11. Vorrichtung (1) nach Anspruch 10, wobei der Aussonderungs- oder Auffangbereich (8) auf einem Höhenniveau angeordnet ist, welches Höhenniveau unterhalb eines Höhenniveaus des Aufnahmeplatzes (2) und/oder unterhalb eines Höhenniveaus des Ablageplatzes (7) angeordnet ist.

12. Vorrichtung (1) nach einem der Ansprüche 9 bis 11, wobei dem Aufnahmeplatz (2) eine Einstelleinrichtung zur Einstellung eines Höhenniveaus des Aufnahmeplatzes (2) zugeordnet ist.

13. Vorrichtung (1) nach einem der Ansprüche 9 bis 11, wobei der Lagengreifer (3) ein verfahrgesteuerter Sauggreifer ist, welcher mit einer Saugfläche für die Leerdosen (21) der Leerdosenlage (20) ausgestattet ist.

## Claims

1. A method for handling layers of empty cans (20), which layers of empty cans (20) have a plurality of regularly arranged empty cans (21) with openings (22) at the top and lateral shell surfaces (23),
which layers of empty cans (20) are staged at a receiving location (2),
in which method all empty cans (21) of a layer of empty cans (20) are suctioned and held in a suctioned manner by applying negative pressure to the openings (22) at the top sides of the empty cans (21),
wherein the suctioning negative pressure is increased up to a threshold value,
**characterised in that**
the compressive forces of intact empty cans (21) acting on the shell surfaces (23) of the empty cans (21) at the threshold value are compensated for by their inherent stability at least so far that the shell surfaces (23) deform elastically back to their original contour after the negative pressure is undone or reduced.

2. The method according to claim 1, wherein mechanically intact and/or undeformed empty cans (21) continue to be held in a suctioned manner up to the threshold value of the negative pressure, while defective empty cans (21) are plastically deformed before or when the threshold value is reached.

3. The method according to claim 1, wherein mechanically intact and/or undeformed empty cans (21) continue to be held in a suctioned manner up to the threshold value of the negative pressure, while defective empty cans (21) implode before or when the threshold value is reached and thereby disengage from the layer of empty cans (20).

4. The method according to one of the previous claims, wherein the mechanically intact and/or undeformed empty cans (21) being held in a suctioned manner are fed to a depositing location (7) and/or to further processing.

5. The method according to one of the previous claims, wherein the layer of empty cans (20) is suctioned and held in a suctioned manner with a first suction pressure, wherein the suctioned layer of empty cans (20) is arranged above a collection container (13), whereupon the suctioning negative pressure is increased to the threshold value, wherein defective empty cans (21) disengage from the layer of empty cans (20) and fall into the collection container (13), wherein the mechanically intact and/or undeformed empty cans (21) that continue to be held in a suctioned manner are fed to further processing.

6. The method according to one of the previous claims, wherein the layer of empty cans (20) being held in a suctioned manner is lifted up in order to isolate the defective empty cans (21) for removal from the production process or
wherein the layer of empty cans (20) being held in a suctioned manner is guided over a discharge area or collection area (8) after the threshold value has been set, which discharge area or collection area (8) is **characterised by** a lower height level, wherein defective empty cans (21) fall into the discharge area or collection area (8) and are removed from the production process.

7. The method according to one of the claims 1 to 5, wherein the layer of empty cans (20) being held in a suctioned manner is guided perpendicular to a transport direction (TR) over a discharge area or collection area (8) after the threshold value has been set, which discharge area or collection area (8) is **characterised by** a lower height level, wherein defective empty cans (21) fall into the discharge area or collection area (8), and wherein the mechanically intact and/or undeformed empty cans (21) being held in a suctioned manner are subsequently fed in transport direction (TR) to further processing.

8. The method according to one of the claims 6 or 7, in which it is detected if a layer of empty cans (20) held in a suctioned manner comprises only mechanically intact and/or undeformed empty cans (21),
wherein such a layer of empty cans (20) is fed directly to the depositing location (7) and/or to further processing without a detour via the discharge area or collection area (8), or
wherein such a layer of empty cans (20) is fed to the depositing location (7) and/or to further processing without a time delay in order for defective empty cans (21) to be removed.

9. An apparatus (1) for handling layers of empty cans (20), which layers of empty cans (20) have a plurality of regularly arranged empty cans (21) with openings (22) at the top and lateral shell surfaces (23), the apparatus (1) comprising
a receiving location (2), at which the layers of empty cans (20) are staged,
a depositing location (7) for the empty cans (21), which depositing location (7) is formed by a discharging transport device, or to which depositing location (7) a discharging transport device is assigned,
a layer gripper (3), which layer gripper (3) is equipped to seize the layer of empty cans (20) at the receiving location (2) in a suctioned manner, and which layer gripper (3) is equipped to move the layer of empty cans (20) to and release it at the depositing location (7),
a control device (30) for the control of the layer gripper (3) and of other machine components
**characterised in that**
the apparatus (1) comprises a device for receiving and/or removing defective empty cans,
wherein the control device (30) comprises a programme for the control of the layer gripper (3), which programme provides following method parameters:
- seizing a layer of empty cans (20) at the receiving location (2) by applying a first negative pressure;
- increasing the negative pressure to a predefined threshold value, at which threshold value the compressive forces of intact empty cans (21) acting on the shell surfaces (23) of the empty cans (21) are compensated for by their inherent stability at least so far that the shell surfaces (23) deform elastically back to their original contour after the negative pressure is undone or reduced.
- release of mechanically intact and/or undeformed empty cans (21) at the depositing location (7).

10. The apparatus (1) according to claim 9, wherein the device for receiving and/or removing defective empty cans (21) comprises a discharge area or collection area (8), which discharge area or collection area (8) is formed in the area of the receiving location (2), or
which discharge area or collection area (8) is formed adjacent to the receiving location (2), or
which discharge area or collection area (8) is formed between the receiving location (2) and the depositing location (7).

11. The apparatus (1) according to claim 10, wherein the discharge area or collection area (8) is arranged at a height level, which height level is arranged below a height level of the receiving location (2) and/or below a height level of the depositing location (7).

12. The apparatus (1) according to one of the claims 9 to 11, wherein a setting device for setting a height level of the receiving location (2) is assigned to the receiving location (2).

13. The apparatus (1) according to one of the claims 9 to 11, wherein the layer gripper (3) is a movement-controlled suction gripper, which is equipped with a suction surface for the empty cans (21) of the layer of empty cans (20).

## Revendications

1. Procédé de manipulation de couches de boîtes vides (20), lesquelles couches de boîtes vides (20) comprennent une pluralité de boîtes vides (21) qui sont disposées régulièrement et présentent des ouvertures supérieures (22) et des surfaces latérales (23) latérales,
lesquelles couches de boîtes vides (20) sont fournies sur un emplacement de réception (2),
dans lequel procédé toutes les boîtes vides (21) d'une couche de boîtes vides (20) sont aspirées et maintenues par aspiration en appliquant une pression négative aux ouvertures supérieures (22) des boîtes vides (21),
dans lequel la pression négative d'aspiration est augmentée jusqu'à une valeur limite,
**caractérisé par le fait que**
à la valeur limite, les forces de pression de boîtes vides (21) intactes, qui agissent sur les surfaces latérales (23) des boîtes vides (21), sont compensées par la stabilité inhérente de celles-ci au moins dans la mesure où les surfaces latérales (23) se déforment élastiquement de manière à reprendre leur contour initial une fois la pression négative supprimée ou réduite.

2. Procédé selon la revendication 1, dans lequel des boîtes vides (21) mécaniquement intactes et/ou non déformées continuent d'être maintenues par aspiration jusqu'à la valeur limite de la pression négative, tandis que des boîtes vides (21) défectueuse sont déformées plastiquement avant que ou lorsque la valeur limite soit/est atteinte.

3. Procédé selon la revendication 1, dans lequel des boîtes vides (21) mécaniquement intactes et/ou non déformées continuent d'être maintenues par aspiration jusqu'à la valeur limite de la pression négative, tandis que des boîtes vides (21) défectueuse implosent avant que ou lorsque la valeur limite soit/est atteinte, et se détachent ainsi de la couche de boîtes vides (20).

4. Procédé selon l'une quelconque des revendications précédentes, dans lequel les boîtes vides (21) mécaniquement intactes et/ou non déformées qui sont maintenues par aspiration sont amenées à un emplacement de dépôt (7) et/ou à un traitement ultérieur.

5. Procédé selon l'une quelconque des revendications précédentes, dans lequel la couche de boîtes vides (20) est aspirée au moyen d'une première pression d'aspiration et est maintenue par aspiration, dans lequel la couche de boîtes vides (20) aspirée est disposée au-dessus d'un récipient collecteur (13), ce après quoi la pression négative d'aspiration est augmentée jusqu'à la valeur limite, dans lequel des boîtes vides (21) défectueuses se détachent de la couche de boîtes vides (20) et tombent dans le récipient collecteur (13), dans lequel les boîtes vides (21) mécaniquement intactes et/ou non déformées qui sont toujours maintenus par aspiration sont amenées au traitement ultérieur.

6. Procédé selon l'une quelconque des revendications précédentes, dans lequel les couches de boîtes vides (20) maintenues par aspiration sont soulevées afin d'isoler les boîtes vides (21) défectueuses pour les retirer du processus de production ou
dans lequel la couche de boîtes vides (20) maintenue par aspiration est guidée sur une zone de tri ou de collecte (8) après avoir réglé la valeur limite, laquelle zone de tri ou de collecte (8) est **caractérisée par** un niveau de hauteur plus faible, dans lequel des boîtes vides (21) défectueuses tombent dans la zone de tri ou de collecte (8) et sont retirées du processus de production.

7. Procédé selon l'une quelconque des revendications 1 à 5, dans lequel, après avoir réglé la valeur limite, la couche de boîtes vides (20) maintenue par aspiration est guidée perpendiculairement à une direction de transport (TR) sur une zone de tri ou de collecte (8), laquelle zone de tri ou de collecte (8) est **caractérisée par** un niveau de hauteur plus faible, dans lequel des boîtes vides (21) défectueuses tombent dans la zone de tri ou de collecte (8), et dans lequel les boîtes vides (21) mécaniquement intactes et/ou non déformées qui sont maintenues par aspiration sont ensuite amenées dans la direction de transport (TR) au traitement ultérieur.

8. Procédé selon l'une quelconque des revendications 6 ou 7, dans lequel on reconnaît qu'une couche de boîtes vides (20) maintenue par aspiration ne comprend que des boîtes vides (21) mécaniquement intactes et/ou non déformées,
dans lequel une telle couche de boîtes vides (20) est amenée directement à l'emplacement de dépôt (7) et/ou au traitement ultérieur sans détour par la zone de tri ou de collecte (8), ou
dans lequel une telle couche de boîtes vides (20) est amenée à l'emplacement de dépôt (7) et/ou au traitement ultérieur sans aucun délai afin d'éliminer des boîtes vides (21) défectueuses.

9. Dispositif (1) de manipulation de couches de boîtes vides (20), lesquelles couches de boîtes vides (20) comprennent une pluralité de boîtes vides (21) qui sont disposées régulièrement et présentent des ouvertures supérieures (22) et des surfaces latérales (23) latérales, le dispositif (1) comprenant
un emplacement de réception (2) où sont fournies les couches de boîtes vides (20),
un emplacement de dépôt (7) pour les boîtes vides (21), lequel emplacement de dépôt (7) est formé par un dispositif de transport qui évacue ou auquel emplacement de dépôt (7) est associé un dispositif de transport qui évacue,
un dispositif de préhension de couche (3), lequel dispositif de préhension de couche (3) est équipé pour saisir par aspiration la couche de boîtes vides (20) à l'emplacement de réception (2), et lequel dispositif de préhension de couche (3) est équipé pour déplacer la couche de boîtes vides (20) vers l'emplacement de dépôt (7) et pour y libérer celle-ci,
un dispositif de commande (30) pour commander le dispositif de préhension de couche (3) et d'autres composants de machine,
**caractérisé par le fait que**
le dispositif (1) comprend un dispositif de réception et/ou d'évacuation de boîtes vides défectueuses,
dans lequel le dispositif de commande (30) comprend un programme pour commander le dispositif de préhension de couche (3), lequel programme prévoit les paramètres de procédé suivants:
- saisir une couche de boîtes vides (20) à l'emplacement de réception (2) en appliquant une première pression négative;
- augmenter la pression négative à une valeur limite prédéfinie; à laquelle valeur limite, les forces de pression de boîtes vides (21) intactes, qui agissent sur les surfaces latérales (23) des boîtes vides (21), sont compensées par la stabilité inhérente de celles-ci au moins dans la mesure où les surfaces latérales (23) se déforment élastiquement de manière à reprendre leur contour initial une fois la pression négative supprimée ou réduite;
- libérer des boîtes vides (21) mécaniquement intactes et/ou non déformées, à l'emplacement de dépôt (7).

10. Dispositif (1) selon la revendication 9, dans lequel le dispositif de réception et/ou d'évacuation de boîtes vides (21) défectueuses comprend une zone de tri ou de collecte (8), laquelle zone de tri ou de collecte (8) est réalisée dans la zone de l'emplacement de réception (2), ou
laquelle zone de tri ou de collecte (8) est formée de manière voisine de l'emplacement de réception (2), ou
laquelle zone de tri ou de collecte (8) est réalisée entre l'emplacement de réception (2) et l'emplacement de dépôt (7).

11. Dispositif (1) selon la revendication 10, dans lequel la zone de tri ou de collecte (8) est disposée à un niveau de hauteur, lequel niveau de hauteur est disposé en dessous d'un niveau de hauteur de l'emplacement de réception (2) et/ou en dessous d'un niveau de hauteur de l'emplacement de dépôt (7).

12. Dispositif (1) selon l'une quelconque des revendications 9 à 11, dans lequel à l'emplacement de réception (2) est associé un dispositif de réglage destiné à régler un niveau de hauteur de l'emplacement de réception (2).

13. Dispositif (1) selon l'une quelconque des revendications 9 à 11, dans lequel le dispositif de préhension de couche (3) est une ventouse à déplacement commandé qui est équipée d'une surface d'aspiration pour les boîtes vides (21) de la couche de boîtes vides (20).
